# EUROPÄISCHE PATENTSCHRIFT

(11) **EP 0 336 263 B1**
(45) Veröffentlichungstag und Bekanntmachung des Hinweises auf die Patenterteilung: **08.03.1995**
(21) Anmeldenummer: 89105460.3
(22) Anmeldetag: 28.03.1989
(51) Int. Cl.: C07C 43/11, C07C 41/03, C07C 41/34

(54) **Verfahren zur Herstellung von Fettalkoholpolyglykolethern mit verringertem Rest-EO-Gehalt bzw. Rest-PO-Gehalt**
Process for the preparation of fatty alcohol polyglycol ethers with reduced residual EO or PO content
Procédé pour la préparation d'éthers polyglycoliques d'alcools gras ayant un contenu d'EO ou de PO diminué

(30) Priorität: 02.04.1988 DE 3811319
(43) Veröffentlichungstag der Anmeldung: 11.10.1989
(73) Patentinhaber: Henkel Kommanditgesellschaft auf Aktien, 40191 Düsseldorf (DE)
(72) Erfinder: Schmid, Karl, Dr., D-4020 Mettmann (DE); Meffert, Alfred, Dr., D-4019 Monheim (DE); Friedrich, Klaus, Dr., D-4000 Düsseldorf (DE); Langen, Michael, D-4010 Hilden (DE); Herrmann, Klaus, D-4019 Monheim (DE)

(56) Entgegenhaltungen:
- DE-A- 1 240 062
- FR-A- 2 508 917
- GB-A- 1 074 249
- US-A- 4 443 634

## Beschreibung

Oberflächenaktive Fettalkoholpolyglykolether aus der Umsetzung längerkettiger Alkohole mit Ethylenoxid und/oder Propylenoxid sind seit Jahrzehnten bekannt. Sie sind wichtige Vertreter der Klasse der nichtionischen Tenside und in der Kosmetik, der Textiltechnik, insbesondere der Textilwäsche und/oder in allgemeinen Anwendungsgebieten des Haushalts und der Technik weit verbreitet.

Die Ethoxylierung bzw. Propoxylierung solcher Alkoholfunktionen wird bei erhöhten Temperaturen und Drucken in Gegenwart von sauren oder basischen Katalysatoren vorgenommen. Besondere praktische Bedeutung hat hier die Verwendung von basischen Verbindungen der Alkalimetalle in all den Fällen, in denen die zu ethoxylierenden Ausgangsverbindungen im wesentlichen neutrale Reaktion zeigen. Als basische Katalysatoren sind hier in erster Linie zu nennen: Natrium- und/oder Kaliumalkoxylate wie Natrium- bzw. Kaliummethylat oder die entsprechenden Ethylate. Häufig eingesetzte basische Alkalimetallkatalysatoren sind auch Natrium- bzw. Kaliumhydroxid.

In jüngster Zeit richtet sich die Aufmerksamkeit für den praktischen Einsatz dieser nichtionischen Tenside - zusätzlich zur Erfüllung der Anforderungen des tensidischen Wirkstoffspektrums - auf einen bestimmten Parameter dieser Hilfsstoffe, nämlich auf ihren Restgehalt an nicht umgesetztem Ethylenoxid. So soll gemäß einer Empfehlung des Bundesgesundheitsamtes der Bundesrepublik Deutschland (siehe Bundesgesundheitsblatt 29 (1986), Seite 21) der Gehalt an freiem EO im nichtionischen Tensid den Grenzwert von 1 ppm nicht überschreiten, sofern der Einsatzzweck für die hier betroffenen Verbindungen den intensiven Kontakt mit dem Körper vorsieht. Zur Einstellung derart erniedrigter EO-Restgehalte bedarf es für die bisher in der Großtechnik eingesetzten Ethoxylierungsverfahren unter Einsatz alkalischer Katalysatoren zusätzlicher Reinigungsmaßnahmen. So wird in der deutschen Patentanmeldung DE-A-3708813 der Anmelderin vorgeschlagen, den Restgehalt des Ethoxylierungsproduktes an freiem EO durch Dämpfen auf die geforderten Werte abzusenken.

Entsprechendes gilt für die Propoxylierung. Auch hier werden Propoxylate mit möglichst niedrigen Restgehalten an freiem PO angestrebt.

Aus der **DE-AS 12 40 062** (Hüls) ist ein verfahren zur Herstellung von Anlagerungsprodukten von Ethylen- und/oder Propylenoxid an gesättigte sekundäre Alkohole mit 8 bis 20 Kohlenstoffatomen bekannt, bei dem man zunächst mindestens 50 Mol-% der Alkohole in die Alkoholatform überführt und diese dann in bekannter Weise mit den Alkylenoxiden abreagieren läßt.

Die Erfindung geht von der Aufgabe aus, die an sich bekannte Ethoxylierung von alkoholischen Ausgangskomponenten der geschilderten Art in Gegenwart von basischen Katalysatoren, insbesondere auf Basis basischer Alkalimetallverbindungen, derart zu modifizieren, daß unmittelbar als primäres Reaktionsprodukt Ethoxylate bzw. Propoxylate der geforderten technischen Spezifikation erhalten werden können, die sich zusätzlich durch den geforderten niederen Restgehalt an freiem EO bzw. PO auszeichnen.

Überraschenderweise wurde gefunden, daß beim Arbeiten mit vergleichsweise erhöhten Katalysatorkonzentrationen, die in der Regel oberhalb der heute technisch eingesetzten Katalysatormengen liegen, Reaktionsprodukte der gewünschten Art erhalten werden können. Gemäß der Erfindung ist es darüberhinaus möglich, weitere wichtige Vorteile für die derart hergestellten Reaktionsprodukte einzustellen, wie sie im nachfolgenden ausführlich geschildert werden. Wesentlich ist dabei insbesondere, daß es durch die Lehre der Erfindung möglich wird, die Absenkung des Restgehaltes an freiem EO bzw. PO zu vervirklichen, ohne im jeweiligen Reaktionsprodukt die bewährten, insbesondere netzenden und/oder emulgierenden Stoffeigenschaften substantiell zu ändern oder gar zu mindern.

Gegenstand der Erfindung ist dementsprechend Fettalkohol- ein Verfahren zur Herstellung von Polyglykolethern mit vermindertem Rest-EO- bzw. Rest-PO-Gehalt, bei dem man
a) Fettalkohole mit 8 bis 36 Kohlenstoffatomen in Gegenwart von 0,5 bis 1,5 Gew.-% - bezogen auf die Summe der Einsatzstoffe - basischer Alkalimetallverbindungen als Katalysatoren mit Ethylen- und/oder Propylenoxid umsetzt,
b) die basischen Alkalimetallverbindungen anschließend mit organischen und/oder anorganischen Säuren in Gegenwart von im Reaktionsprodukt dispergierten, feinteiligen Feststoffen neutralisiert und
c) die ausgefällte Salzphase abfiltriert.

In der bevorzugten Ausführungsform der Erfindung wird die Alkoxylierung mit einer Mindestkonzentration von 0,8 Gew.-% des basischen Katalysators durchgeführt. Erfindungsgemäß wird es dabei möglich, den Rest-EO-Gehalt im primär anfallenden Reaktionsprodukt auf Werte von höchstens etwa 0,5 ppm einzustellen, wobei in der Regel die Restgehalte an freiem EO im primären Reaktionsprodukt unter der zur Zeit gegebenen Nachweisgrenze liegen, die mit etwa 0,1 ppm angegeben werden kann. Die entsprechenden Restgehalte an freiem PO liegen unter 5 ppm.

Der Stand der Technik hat sich ausführlich mit der Herstellung von Alkylpolyglykolether-Reaktionsprodukten unter Einsatz erhöhter Mengen alkalischer Katalysatoren der hier betroffenen Art beschäftigt. Es ist bekannt, daß insbesondere durch Verwendung erhöhter Katalysatormengen Reaktionsprodukte mit eingeengter Homologenverteilung anfallen.

Die bei der Alkoholalkoxylierung gebildeten Alkoxylatreaktionsprodukte werden zwar der Einfachheit halber stöchiometrisch formuliert - etwa in der Art C₁₂/C₁₄-Fettalkohol + 9 EO - in Wirklichkeit liegt aber als Reaktionsprodukt das Gemisch von homologen Alkoxylaten von vergleichsweise niedrig bis hochalkoxylierten Reaktionsprodukten vor. Die jeweilige Homologenverteilung folgt Gesetzmäßigkeiten, die sich aus der Struktur des eingesetzten Alkohols und den Bedingungen bei der Alkoxylierung ableiten. Eine wichtige Rolle spielt in diesem Zusammenhang Art und Menge des verwendeten Katalysators. Wichtige anwendungstechnische Eigenschaften wie Netzvermögen, Waschvermögen, Reinigungsvermögen, Emulgiervermögen, entschäumende Wirkung und dergleichen werden durch die jeweilige Homologenverteilungskurve der Reaktionsprodukte bestimmt. Die heute auf dem Markt befindlichen Handelsprodukte bringen hier ein optimiertes Leistungsniveau, das als Grundstandard der Praxis für eventuelle Abwandlungen in der Homologenverteilung entsprechender Reaktionsprodukte zu berücksichtigen und möglichst nicht zu ändern ist. Zur Beeinflussung der anwendungstechnischen Eigenschaften - insbesondere also des Netz- bzw. Emulgiervermögens - beim Arbeiten mit erhöhten Katalysatorkonzentrationen finden sich im einschlägigen Stand der Technik unterschiedliche Aussagen. Teilweise werden unerwünschte Änderungen postuliert, gemäß anderen Aussagen sollen schwerwiegende Änderungen hier nicht eingetreten sein.

Die Erfindung hat sich die Aufgabe gestellt, Reaktionsgemische der beschriebenen Art herzustellen, die in ihren anwendungstechnischen Eigenschaften dem heute von der Praxis geforderten Standard voll entsprechen; gleichzeitig soll jedoch die geforderte Absenkung des Restgehalts an freiem EO bzw. PO sichergestellt sein.

In einer besonderen Ausführungsform verbindet die Lehre der Erfindung die Erfüllung dieses Ziels mit einer weiteren wichtigen Verbesserung für ausgewählte Einsatzmaterialien und daraus herstellbare polyalkoxylierte Reaktionsprodukte.

Die Erfindung hat sich hier die zusätzliche Aufgabe gestellt, Alkylpolyglykolether-Reaktionsgemische herzustellen, die in ihren anwendungstechnischen Eigenschaften dem heute von der Praxis geforderten Standard voll entsprechen. Gleichzeitig soll jedoch das Kälteverhalten der Reaktionsgemische gegenüber den vergleichbaren üblichen Produkten substantiell verbessert sein. In der Praxis bedeutet das, daß insbesondere bei den entsprechenden Reaktionsprodukten mit Fließfähigkeit im Bereich der Raumtemperatur oder nur schwach darüber eine verbesserte Flüssigkeitscharakteristik und insbesondere der Zustand der Klarflüssigkeit verwirklicht sein soll. Bedeutung hat dieser Gesichtspunkt insbesondere für EO-Kondensationsprodukte.

Die Erfindung will dabei bewußt an den für die praktische Anwendung bewährten basischen Alkalimetallverbindungen als Katalysatoren festhalten. Durch eine - an sich bekannte - Erhöhung der Katalysatorkonzentration für die Alkoholethoxylierung soll - zusätzlich zur Absenkung des Rest-EO-Gehalts - eine möglichst durchgreifende Verbesserung des Kälteverhaltens im Reaktionsprodukt erzielt werden, ohne daß gleichzeitig eine substantielle Änderung der anwendungstechnischen Eigenschaften ausgelöst wird.

Bei den Arbeiten zur Verwirklichung dieses Prinzips hat sich gezeigt, daß eine beträchtliche verfahrenstechnische Schwierigkeit auftritt. In an sich bekannter Weise schlägt das Verfahren der Erfindung vor, die Menge des basischen Alkalimetallkatalysators bei der Ethoxylierungsreaktion heraufzusetzen, um eine Einengung der Homologen-Verteilung und insbesondere die Beseitigung störender Anteile im Bereich der höheren und höchsten Ethoxylatprodukte zu bewirken. Dabei müssen jedoch die basischen Alkalimetallkatalysatoren in solcher Menge eingesetzt werden, daß eine beträchtliche verfahrenstechnische Schwierigkeit für das großtechnische Verfahren entsteht.

Mit alkalischen Katalysatoren hergestellte Alkyl- oder Alkylarylalkoxylate müssen direkt nach der Herstellung auf pH-Werte im Bereich zwischen etwa 6,5 und 7,5 neutralisiert werden, da sie sich sonst unter Einwirkung von Luftsauerstoff stark verfärben. Besonders empfindlich sind in dieser Hinsicht Alkoxylate auf Basis ungesättigter Fettalkohole, zum Beispiel auf Basis Oleylalkohol. Die Neutralisation des Katalysators im Reaktionsprodukt wird mit organischen und/oder anorganischen Säuren durchgeführt. Als Beispiele seien genannt Gluconsäure, Glykolsäure, Essigsäure, Ameisensäure, Benzoesäure, Milchsäure, Oxalsäure, Citronensäure, Propionsäure, Phosphorsäure, Methansulfonsäure und/oder Diglykolsäure. Stark korrosive Säuren wie Schwefelsäure oder Salzsäure haben in der Praxis keine Bedeutung.

Bei der Neutralisationsreaktion reagieren die zugesetzten Säuren mit dem im Alkoxylat enthaltenen Kalium- bzw. Natriumalkoxylat zum entsprechenden Salz. Diese Salze besitzen im Reaktionsprodukt nur eine beschränkte Löslichkeit. Wird die Katalysatormenge für die technische Herstellung des Alkoxylats so hoch gewählt, daß bei der Neutralisation die Salzlöslichkeit nicht mehr gewährleistet ist, so entstehen im Reaktionsprodukt zunächst Trübungen und in zunehmendem Ausmaß Salzausfällungen. In der Praxis wird dementsprechend bisher die eingesetzte Katalysatormenge so bemessen, daß das mit der zur Neutralisation benötigten Menge Säure entstehende Salz im Alkoxylat-Reaktionsprodukt gerade noch löslich ist. Als Grenzwert ist hier etwa ein Betrag von etwa 0,5 Gew.-% Natriummethylat - bezogen auf die Gesamtmenge von Alkohol und Ethylenoxid bzw. Propylenoxid - bzw. äquivalent entsprechende Mengen anderer basischer Alkalimetallverbindungen anzusehen. Wird die Katalysatormenge darüber hinaus erhöht, so bildet sich bei der Neutralisation mit den angegebenen Säuren ein Salz, das aus dem Alkoxylat als zähklebrige Masse ausfällt und sich sowohl im Neutralisationsbehälter am Rührwerk und an den Behälterwandungen als auch in den Rohrleitungen, in denen das Alkoxylat zur Filtration geführt wird, niederschlägt. In kurzer Frist tritt damit eine so starke Verschmutzung aller Innenbereiche der hier betroffenen Apparaturen und Leitungen auf, daß der Verfahrenszyklus unterbrochen werden muß. Es werden kosten- und zeitaufwendige Reinigungsmaßnahmen erforderlich. Die daraus anfallenden Belastungen sind so beträchtlich, daß die Reaktionsprodukte aus wirtschaftlichen Gründen uninteressant geworden sind.

Die Lösung der erfindungsgemäßen Aufgabenstellungen geht von der überraschenden Erkenntnis aus, daß man bei einem begrenzten Anheben der Menge der basischen Alkalimetallkatalysatoren nicht nur den Rest-EO-Gehalt bzw. den Rest-PO-Gehalt im gewünschten Ausmaß senken, sondern darüberhinaus substantielle Verbesserungen der die Kältestabilität charakterisierenden Stoffparameter einstellen kann, ohne in unerwünschter Weise die anwendungstechnischen Eigenschaften wie Wasch- bzw. Netzvermögen, Emulgiervermögen und dergleichen zu beeinträchtigen. Diese Verbesserung des Kälteverhaltens ist vor allem für die Ethoxylate von Bedeutung, da diese in der Regel einen höheren Stockpunkt aufweisen als entsprechende Propoxylate. Die Lehre der Erfindung muß dabei allerdings die Menge des basischen Katalysators über den Grenzbetrag hinaus erhöhen, der die Löslichkeit des durch Neutralisation anfallenden Salzes im Ethoxylierungsprodukt kennzeichnet. Zur Lösung dieser Schwierigkeit schlägt das erfindungsgemäße Verfahren vor, die Katalysatorneutralisation in Gegenwart von feinteiligen Feststoffen vorzunehmen, die im Reaktionsgemisch möglichst homogen verteilt sind.

Im Verfahren der Erfindung wird dann mit derart erhöhten Katalysatorkonzentrationen gearbeitet, daß in der Neutralisationsstufe eine Salzfällung als ungelöste Feststoffphase auftritt, wobei man den Neutralisationsschritt dergestalt durchführt, daß die Ausfällung der Salzphase in Gegenwart von im Reaktionsprodukt dispergierten feinteiligen Feststoffen vorgenommen wird. Überraschenderweise hat sich gezeigt, daß durch diese Maßnahme Verkrustungen an Apparate und/oder Leitungs-Innenteilen vollständig verhindert werden können und daß weiterhin die Filtration des Reaktionsproduktes in konventionellen Verfahren ohne Schwierigkeiten möglich ist. Ohne Einbuße an verfahrenstechnischen Vorteilen wird das mit erfindungsgemäß die Herstellung von Alkylpolyglykolethern im Sinne der zuvor geschilderten erfindungsgemäßen Zielsetzung möglich.

Als fein verteilte Feststoffe zur Kristallisationshilfe werden erfindungsgemäß bevorzugt übliche Filterhilfsmittel anorganischer und/oder organischer Natur eingesetzt. Diese Filterhilfsmittel werden in dem Reaktionsgemisch aus der Alkoxylierung dispergiert, bevor eine Auskristallisation der sich bildenden Salzphase stattfindet. In der zweckmäßigsten Ausführungsform werden die Filterhilfsmittel im Reaktionsprodukt fein verteilt, bevor mit dem Säurezusatz begonnen wird.

Als Filterhilfsmittel sind sowohl anorganische Materialien, insbesondere Kieselgur, als auch organische bekannte Filterhilfsmittel geeignet. Organische Materialien können zum Beispiel sein Holzmehl und/oder feinteilige Zellulose. Filterhilfsmittel auf dieser organischen Basis sind zum Beispiel unter ihren geschützten Handelsbezeichnungen "Arbocel" und "Lignocell" bekannt.

Ein Vorteil der Verwendung von organischen Filterhilfsmitteln besteht darin, daß sie zusammen mit dem aus dem Alkoxylat erhaltenen Salz (beispielsweise Natriumgluconat) verbrannt werden können, während das mit diesen Salzen verunreinigte anorganische Filterhilfsmittel auf einer Deponie gelagert werden müßte.

Es kann allerdings zweckmäßig sein, Mischungen aus organischen und anorganischen Filterhilfsmitteln einzusetzen, wobei hier üblicherweise Mischungsverhältnisse der jeweiligen Bestandteile im Bereich von 3 : 1 bis 1 : 3 besonders geeignet sind.

Die Menge des Filterhifsmittels für die Salzbildung liegt im allgemeinen im Bereich von etwa 0,3 bis 2 Gew.-% und vorzugsweise im Bereich von etwa 0,5 bis 1 Gew.-%, Gew.-% jeweils bezogen auf die Produktmenge des gebildeten Alkoxylats.

Im erfindungsgemäßen Verfahren kann beispielsweise wie folgt vorgegangen werden: Das die vergleichsweise erhöhte Katalysatormenge enthaltende Reaktionsprodukt, das in an sich bekannter Weise bei Abschluß der Reaktion eine Temperatur von etwa 150 bis 180 °C aufweist, wird nach Beendigung der Alkoxylierung aus dem Druckbehälter in einen Neutralisationsbehälter mit Rührwerk überführt. Hier wird das Reaktionsgemisch unter Rühren auf den Temperaturbereich von etwa 50 bis 110 °C, vorzugsweise etwa 80 bis 100 °C, abgekühlt und das Filterhilfsmittel dem Produkt zugegeben und darin möglichst homogen verteilt. Dann wird die Neutralisation mit der gewählten Säure vorgenommen. Nach Abschluß der Neutralisation wird das Alkoxylat, in dem Filterhilfsmittel und gebildetes Salz homogen verteilt sind, zur Filtrationsstufe gepumpt. Die Filtration kann sowohl über Durchflußfilter (Filterkerzen, Seitzfilter udgl.) als auch über Filterpressen und über Drehfilter erfolgen. Besonders salzarme Alkoxylate erhält man, wenn die unter Stickstoff erfolgende Filtration bei höheren Temperaturen als 80 °C durchgeführt wird.

Wird nach diesem Verfahren der Neutralisation und Filtration gearbeitet, so sind weder im Neutralisationsbehälter an den Wandungen oder am Rührwerk noch in den Rohrleitungen oder Pumpen irgendwelche zähklebrigen Rückstände von Salz feststellbar.

In einer besonderen Ausführungsform des erfindungsgemäßen Verfahrens wird das Alkoxylat-Reaktionsprodukt im Neutralisationsbehälter - mit dem darin homogen verteilten Filterhilfsmittel - zunächst durch Säurezusatz nur anteilsweise, beispielsweise bis auf einen pH-Wert von ca. 8 neutralisiert. Anschließend wird das Reaktionsprodukt insbesondere durch Zugabe von Wasserstoffperoxid, beispielsweise in Mengen von etwa 0,1 bis 1 Gew.-%, - bezogen auf das Alkoxylat - gebleicht. Erst nach der Bleiche wird die Neutralisationsreaktion durch Zugabe weiterer Säuremengen abgeschlossen und dabei der End-pH im Bereich von etwa 6,5 bis 7,5 eingestellt. Dann wird das Alkoxylat filtriert.

Die im erfindungsgemäßen Verfahren eingesetzte Katalysatormenge an basischen Alkalimetallverbindungen liegt grundsätzlich höher als es in der Praxis bisher als zweckmäßig angesehen wurde. Die Katalysatormengen liegen üblicherweise oberhalb des Grenzwertes von etwa 0,5 Gew.-%, berechnet als Natriummethylat und bezogen auf das Gesamtgewicht von eingesetztem Alkohol und Ethylenoxid. Werden andere alkalische Katalysatoren eingesetzt, beispielsweise also Kaliumalkoholat oder Natrium- bzw. Kaliumhydroxyd, dann werden äquivalente Mengenverhältnisse des Katalysators gewählt.

Die Festlegung der jeweils bestimmten Katalysatormenge wird durch die Struktur des eingesetzten Alkohols mitbestimmt. Alkohole mit stöchiometrisch nicht gehinderter Hydroxylgruppe, insbesondere also lineare Alkohole etwa von der Art der Fettalkohole oder der Ziegleralkohole bzw. auch verzweigte Alkohole mit sterisch nicht gehinderter Primärer Hydroxylgruppe erfordern für die Einstellung des erfindungsgemäß angestrebten Ergebnisses der erhöhten Kältestabilität bei unveränderten anwendungstechnischen Eigenschaften üblicherweise Mengen an Natriummethylatkatalysator im Bereich von etwa 0,5 bis 1,5 Gew.-%, bevorzugt im Bereich von etwa 0,8 bis 1,1 Gew.-% - jeweils bezogen auf die Gesamtmenge von eingesetztem Alkohol, Ethylenoxid und/oder Propylenoxid.

Durch die Verwendung derart erhöhter Katalysatormengen und durch Einsatz der geschilderten technischen Problemlösung zur Entfernung der dabei dann zwangsläufig gebildeten klebrigen Reaktionssalze aus der Neutralisationsstufe erhält man Alkoxylate, die im Vergleich zu den nach bisher in der Praxis üblichen Verfahren mit niedrigerer Katalysatormenge hergestellten Produkten im Falle der Ethoxylate den abgesenkten Rest-EO-Gehalt und gleichzeitig ein wesentlich besseres Kälteverhalten bzw. im Falle der Propoxylate den abgesenkten Rest-PO-Gehalt aufweisen, ohne Beeinträchtigung oder sogar unter Verbesserung aller anderen anwendungstechnisch relevanten Eigenschaften wie Waschvermögen, Reinigungskraft und Emulgiervermögen. Wichtig ist diese Verbesserung insbesondere für die Handhabung von Nonionics der hier geschilderten Art, die nach den bisherigen Herstellungsverfahren ganz oder teilweise eingedickt sind bzw. zur Ausbildung von Feststoffanteilen, insbesondere beim Stehen unter Raumtemperatur, neigen. Erfindungsgemäß wird es möglich, diese Stoffgemische in ihrem Kälteverhalten so zu verbessern, daß bei Raumtemperatur fließfähige und bevorzugt klare Homologengemische vorliegen. Die Bestimmung des Kälteverhaltens kann dabei meßtechnisch in bekannter Weise durch Bestimmung des Kältetrübungspunktes (DIN ISO 3015), des Stockpunktes (DIN ISO 3016) sowie des Kälteklarpunktes erfolgen. Der Kälteklarpunkt wurde dabei in den nachfolgenden Beispielen wie folgt bestimmt: 100 g des zu prüfenden Produktes werden auf minus 40 °C abgekühlt, so daß die Probe in jedem Fall fest war. Danach ließ man die Probe, in der sich ein Thermometer befand, bei Raumtemperatur auftauen und bestimmte die Temperatur, bei der das Produkt gerade klarflüssig wurde (Kälteklarpunkt).

Als Einsatzmaterialien mit alkoxylierbarer Alkoholfunktion kommen für das erfindungsgemäße Verfahren Fettalkohole mit 8 bis 36 Kohlenstoffatomen in Betracht.

Für die Praxis bedeutungsvoll ist insbesondere der Kohlenstoffkettenbereich von etwa C₁₀ bis C₂₄.

In den nachfolgenden Beispielen und Vergleichsbeispielen werden alle Fettalkoholethoxylate bzw. Fettalkohol-EOₓ-PO_{y}-Addukte wie folgt hergestellt:

Für die Herstellung von Fettalkoholethoxylaten werden die jeweils in den Beispielen angegebenen Mengen an Fettalkohol, Ethylenoxid und Katalysator in einem Autoklaven bei 170 bis 180 °C mit einem Reaktionsdruck von 2 bis maximal 5 bar ca. 3 bis 4 Stunden umgesetzt.

Für die Herstellung von Fettalkohol-EOₓ-PO_{y}-Addukten werden zunächst die in den Beispielen angegebenen Mengen an Fettalkohol, Ethylenoxid und Katalysator in einem Autoklaven bei 170 bis 180 °C mit einem Reaktionsdruck von 2 bis maximal 5 bar ca. 3 bis 4 Stunden umgesetzt. Nach Abklingen der Reaktion, was sich in einer starken Abnahme des Reaktionsdruckes bemerkbar macht, werden die in den Beispielen angegebenen Mengen an Propylenoxid in den Autoklaven eingegeben und die Reaktion bei 150 °C bis 170 °C und einem Reaktionsdruck von 2 bis maximal 5 bar für weitere 3 bis 4 Stunden fortgesetzt.

Nach Abschluß der Alkoxylierungsreaktion wird das Reaktionsprodukt (Fettalkoholethoxylat oder Fettalkohol-EOₓ-PO_{y}-Addukt) in einen mit Rührer ausgestatteten Neturalisationsbehälter überführt und 1 Gew.-% Filterhilfsmittel durch Rühren in dem auf ca. 90 °C abgekühlten Produkt homogen verteilt. Dann wird die Neutralisation mit Milchsäure vorgenommen, so daß das Reaktionsprodukt schließlich einen pH-Wert (1 % des Produkts in 99 % deionisiertem Wasser) von 6,5 bis 7,5 aufweist.

Die Bestimmung der Hydrophilie (Trübungspunkt, Trübungstemperatur, Trübungstitrationszahl) des Ethoxylats efolgt nach DIN 53 917.

Die Bestimmung des Restgehaltes an freiem Ethylenoxid und/oder Propylenoxid in den Reaktionsprodukten, wie sie in den Beispielen bzw. Vergleichsbeispielen beschrieben sind, erfolgt durch quantitative Kapillargaschromatographie. Die Nachweisgrenze betrug bei Ethylenoxid kleiner/gleich 0,1 ppm, bei Propylenoxid kleiner/gleich 5 ppm.

### Beispiele

### Vergleichsbeispiel 1

679,9 g Oleylalkohol mit einer Jodzahl von 72 und einer Hydroxylzahl von 212 wurden mit 520,0 g Ethylenoxid und 3,6 g Natriummethylat (30 %ig, in Methanol) (entspricht 0,09 Gew.-% Natriummethylat bezogen auf die Gesamtmenge von Alkohol und Ethylenoxid) umgesetzt. Man erhielt ein Ethoxylat mit folgenden Kennzahlen:

| | |
|---|---|
| OHZ | 119,0 |
| % H₂O | 0,11 |
| Dichte (20 °C) | 0,952 g/cm³ |
| Trübungstemperatur (5 g in 25 g 25 %igem Butyldiglykol) | 67,4 °C |
| pH-Wert (1 %ig) | 7,1 |
| % Polyethylenglykol | 1,2 mit einem Molekulargewicht von 1000 |
| Kälteklarpunkt | > 30 °C |
| Stockpunkt | 10 °C |
| Kältetrübungspunkt | 21 °C |
| Gehalt an freiem Ethylenoxid: | 65 ppm |

### Beispiel 1

667,3 g Oleylalkohol mit einer Jodzahl von 72 und einer Hydroxylzahl von 212 wurden mit 532,7 g Ethylenoxid und 36,0 g Natriummethylat (30 %ig, in Methanol) (entspricht 0,9 Gew.-% Natriummethylat bezogen auf die Gesamtmenge von Alkohol und Ethylenoxid) umgesetzt. Man erhielt ein Ethoxylat mit folgenden Kennzahlen:

| | |
|---|---|
| OHZ | 119,8 |
| % H₂O | 0,06 |
| Dichte (20 °C) | 0,951 g/cm³ |
| Trübungstemperatur (5 g in 25 g 25 %igem Butyldiglykol) | 67,6 °C |
| pH-Wert (1 %ig) | 6,8 |
| % Polyethylenglykol | 2,3 mit einem Molekulargewicht von 400 |
| Kälteklarpunkt | 16 °C |
| Stockpunkt | 2 °C |
| Kältetrübungspunkt | 12 °C |
| Gehalt an freiem Ethylenoxid: | < 0,5 ppm |

### Vergleichsbeispiel 2

413,0 g eines Gemisches von Octanol/Decanol mit einer Hydroxylzahl von 330 wurden mit 587,0 g Ethylenoxid und 7,0 g Natriummethylat (30 %ig, in Methanol) (entspricht 0,21 Gew.-% Natriummethylat bezogen auf die Gesamtmenge von Alkohol und Ethylenoxid) umgesetzt. Aus dem erhaltenen Ethoxylat wurden 9,4 % des nicht umgesetzten Octanol/Decanol abdestilliert. Man erhielt ein Ethoxylat mit folgenden Kennzahlen:

| | |
|---|---|
| OHZ | 127 |
| % H₂O | 0,11 |
| Dichte (70 °C) | 0,954 g/cm³ |
| Trübungspunkt (1 %ig in Wasser) | 58 °C |
| pH-Wert (1 %ig) | 7,2 |
| % Polyethylenglykol | 4,2 |
| Kälteklarpunkt | > 25 °C |
| Stockpunkt | 10 °C |
| Kältetrübungspunkt | 16 °C |
| Gehalt an freiem Ethylenoxid: | 70 ppm |

### Beispiel 2

550,7 g eines Gemisches von Octanol/Decanol mit einer Hydroxylzahl von 330 wurden mit 849,3 g Ethylenoxid und 42,0 g Natriummethylat (30 %ig, in Methanol) (entspricht 0,90 Gew.-% Natriummethylat bezogen auf die Gesamtmenge von Alkoholen und Ethylenoxid) umgesetzt. Aus dem erhaltenen Ethoxylat wurden 9,1 % des nicht umgesetzten Octanol/Decanol abdestilliert. Man erhielt ein Ethoxylat mit folgenden Kennzahlen:

| | |
|---|---|
| OHZ | 123 |
| % H₂O | 0,13 |
| Dichte (70 °C) | 0,958 g/cm³ |
| Trübungspunkt (1 %ig in Wasser) | 64 °C |
| pH-Wert (1 %ig) | 7,1 |
| % Polyethylenglykol | 4,6 |
| Kälteklarpunkt | 18 °C |
| Stockpunkt | 7 °C |
| Kältetrübungspunkt | 10 °C |
| Gehalt an freiem Ethylenoxid: | < 0,5 ppm |

### Vergleichsbeispiel 3

300 g eines Gemisches von Dodecanol/Tetradecanol mit einer Hydroxylzahl von 289 wurden mit 340 g Ethylenoxid, 360 g Propylenoxid und 2,9 g Natriummethylat (30 %ig, in Methanol) (entspricht 0,09 Gew.-% Natriummethylat, bezogen auf die Gesamtmenge von Alkohol, Ethylenoxid und Propylenoxid) umgesetzt. Man erhielt ein Alkoxylat mit einer Hydroxylzahl von 105 und einem Restgehalt an freiem Propylenoxid von 3000 ppm.

### Beispiel 3

300 g eines Gemisches von Dodecanol/Tetradecanol mit einer Hydroxylzahl von 289 wurden mit 340 g Ethylenoxid, 360 g Propylenoxid und 30,0 g Natriummethylat (30 %ig, in Methanol) (entspricht 1,0 Gew.-% Natriummethylat, bezogen auf die Gesamtmenge von Alkohol, Ethylenoxid und Propylenoxid) umgesetzt. Man erhielt ein Alkoxylat mit einer Hydroxylzahl von 103 und einen Restgehalt an freiem Propylenoxid von 2 ppm.

### Vergleichsbeispiel 4

300 g eines Gemisches von Dodecanol/Tetradecanol mit einer Hydroxylzahl von 289 wurden mit 160 g Ethylenoxid, 528 g Propylenoxid und 2,9 g Natriummethylat (30 %ig, in Methanol) (entspricht 0,09 Gew.-% Natriummethylat, bezogen auf die Gesamtmenge von Alkohol, Ethylenoxid und Propylenoxid) umgesetzt. Man erhielt ein Alkoxylat mit einer Hydroxylzahl von 114 und einem Restgehalt an freiem Propylenoxid von 6000 ppm.

### Beispiel 4

300 g eines Gemisches von Dodecanol/Tetradecanol mit einer Hydroxylzahl von 289 wurden mit 160 g Ethylenoxid, 528 g Propylenoxid und 30,0 g Natriummethylat (30 %ig, in Methanol) (entspricht 1,0 Gew.-% Natriummethylat, bezogen auf die Gesamtmenge von Alkohol, Ethylenoxid und Propylenoxid) umgesetzt. Man erhielt ein Alkoxylat mit einer Hydroxylzahl von 111 und einen Restgehalt an freiem Propylenoxid von 4 ppm.

## Patentansprüche

1. Verfahren zur Herstellung von Fettalkoholpolyglycolether mit vermindertem Rest-EO- bzw. Rest-PO-Gehalt, bei dem man
a) Fettalkohole mit 8 bis 36 Kohlenstoffatomen in Gegenwart von 015 bis 1,5 Gew.-% - bezogen auf die Summe der Einsatzstoffe - basischer Alkalimetallverbindungen als Katalysatoren mit Ethylenoxid - und/ oder Propylenoxid umsetzt,
b) die basischen Alkalimetallverbindungen anschließend mit organischen und/oder anorganischen Säuren in Gegenwart von im Reaktionsprodukt dispergierten, feinteiligen Feststoffen neutralisiert und
c) die ausgefällte Salzphase abfiltriert.

2. Verfahren nach Anspruch 1, **dadurch gekennzeichnet,** daß man Fettalkohole mit 10 bis 24 Kohlenstoffatomen einsetzt.

3. Verfahren nach Anspruch 1, **dadurch gekennzeichnet,** daß man die basischen Alkalimetallverbindungen in Mengen von 0,8 bis 1,1 Gew.-% - bezogen auf die Summe der Einsatzstoffe - einsetzt.

4. Verfahren nach Anspruch 1, **dadurch gekennzeichnet,** daß man als basische Alkalimetallverbindung Natriummethylat einsetzt.

5. Verfahren nach Anspruch 1, **dadurch gekennzeichnet**, daß man als feinteilige Feststoffe organische und/oder anorganische Filterhilfsmittel einsetzt, die im Reaktionsprodukt dispergiert werden, bevor eine Auskristallisation der Salzphase auftritt.

6. Verfahren nach Anspruch 1, **dadurch gekennzeichnet,** daß man die feinteiligen Feststoffe in Mengen von 0,3 bis 2 Gew.-% - bezogen auf das gebildete Reaktionsprodukt - einsetzt.

7. Verfahren nach Anspruch 1, **dadurch gekennzeichnet,** daß man die Ausfällung der Salzphase auf der Oberfläche des dispergierten Filterhilfsmittels im Temperaturbereich von 50 bis 110°C vornimmt.

## Claims

1. A process for the production of fatty alcohol polyglycol ethers having a reduced residual EO or residual PO content, in which
a) fatty alcohols containing 8 to 36 carbon atoms are reacted with ethylene oxide and/or propylene oxide in the presence of 0.5 to 1.5% by weight - based on the sum total of starting materials - of basic alkali metal compounds as catalysts,
b) the basic alkali metal compounds are subsequently neutralized with organic and/or inorganic acids in the presence of fine-particle solids dispersed in the reaction product and
c) the salt phase precipitated is filtered off.

2. A process as claimed in claim 1, characterized in that fatty alcohols containing 10 to 24 carbon atoms are used.

3. A process as claimed in claim 1, characterized in that the basic alkali metal compounds are used in quantities of 0.8 to 1.1% by weight - based on the sum total of starting materials.

4. A process as claimed in claim 1, characterized in that sodium methylate is used as the basic alkali metal compound.

5. A process as claimed in claim 1, characterized in that organic and/or inorganic filter aids are used as the fine-particle solids and are dispersed in the reaction product before the salt phase crystallizes out.

6. A process as claimed in claim 1, characterized in that the fine-particle solids are used in quantities of 0.3 to 2% by weight, based on the reaction product formed.

7. A process as claimed in claim 1, characterized in that the salt phase is precipitated on the surface of the dispersed filter aid at a temperature in the range from 50 to 110°C.

## Revendications

1. Procédé pour la préparation d'éthers polyglycoliques d'alcools gras à teneur résiduelle en OE, respectivement à teneur résiduelle en OP diminuée, dans lequel
a) on fait réagir des alcools gras contenant de 8 à 36 atomes de carbone en présence de 0,5 à 1,5% en poids - rapporté à la somme des substances mises en oeuvre - de composés basiques de métaux alcalins comme catalyseurs, avec de l'oxyde d'éthylène et/ou de l'oxyde de propylène,
b) on neutralise ultérieurement les composés basiques de métaux alcalins avec des acides organiques et/ou inorganiques en présence de substances solides à grains fins dispersées dans le produit réactionnel, et
c) on sépare par filtration la phase saline précipitée.

2. Procédé selon la revendication 1, caractérisé en ce qu'on met en oeuvre des alcools gras contenant de 10 à 24 atomes de carbone.

3. Procédé selon la revendication 1, caractérisé en ce qu'on met en oeuvre les composés basiques de métaux alcalins dans des quantités de 0,8 à 1,1% en poids, rapportées à la somme des substances mises en oeuvre.

4. Procédé selon la revendication 1, caractérisé en ce que, comme composé basique de métal alcalin, on met en oeuvre le méthylate de sodium.

5. Procédé selon la revendication 1, caractérisé en ce que, comme substances solides à grains fins, on met en oeuvre des adjuvants de filtres organiques et/ou inorganiques que l'on disperse dans le produit réactionnel avant qu'intervienne une séparation de la phase saline par cristallisation.

6. Procédé selon la revendication 1, caractérisé en ce qu'on met en oeuvre les substances solides à grains fins dans des quantités de 0,3 à 2% en poids, rapportées au produit réactionnel formé.

7. Procédé selon la revendication 1, caractérisé en ce qu'on procède à la précipitation de la phase saline à la surface de l'adjuvant de filtre dispersé dans le domaine de température de 50 à 110°C.
